Europäisches Patentamt

European Patent Office

Office européen des brevets

(19)

(11) **EP 0 648 730 B1**

(12) **EUROPEAN PATENT SPECIFICATION**

(45) Date of publication and mention
of the grant of the patent:
**06.12.2000 Bulletin 2000/49**

(51) Int. Cl.7: **C07C 45/50**, B01D 53/02

(21) Application number: **94307011.0**

(22) Date of filing: **26.09.1994**

(54) **Process for the production of oxo products**

Verfahren zur Herstellung von Oxoverbindungen

Procédé de préparation de composés oxo

(84) Designated Contracting States:
**BE DE ES FR GB IE IT NL SE**

(30) Priority: **30.09.1993 US 129527**
**22.04.1994 US 231546**

(43) Date of publication of application:
**19.04.1995 Bulletin 1995/16**

(73) Proprietor: **THE BOC GROUP, INC.**
**Murray Hill, New Jersey 07974 (US)**

(72) Inventors:
 • **Ramachandran, Ramakrishnan**
 **Allendale, New Jersey 07401 (US)**

 • **Dao, Loc**
 **Bound Brook, New Jersey 08805 (US)**

(74) Representative:
 **Wickham, Michael et al**
 **c/o Patent and Trademark Department**
 **The BOC Group plc**
 **Chertsey Road**
 **Windlesham Surrey GU20 6HJ (GB)**

(56) References cited:
 **EP-A- 0 147 960       EP-A- 0 404 193**
 **GB-A- 1 375 900       US-A- 4 210 426**

## Description

The present invention is directed to a process for producing oxo products, and more particularly to a process in which propylene, carbon monoxide and hydrogen are reacted in the presence of a suitable catalyst to produce oxo products, such as butyraldehyde and n-butyl alcohol, and unreacted propylene is recycled to the reactor.

Butyraldehyde is produced commercially by the hydroformylation reaction of propylene, carbon monoxide and hydrogen over a suitable catalyst. The reaction effluent is comprised of the butyraldehyde product, unreacted propylene, hydrogen and carbon monoxide, and small amounts of byproducts. Similarly, n-butyl alcohol is produced commercially by the hydroformylation-hydrogenation of propylene with carbon monoxide and hydrogen in the presence of a suitable catalyst. According to one commercial process for the production of butyraldehyde, the reaction is conducted on a semi-batch basis in the mixed gas-liquid phase in a stirred reactor at a temperature of about 130°C. and an absolute pressure of about 14 atmospheres. The reaction is strongly exothermic and part of the heat of reaction is removed by heat exchange and the remainder is used to evaporate the reaction products so that they can be easily removed from the reactor as a gas stream, thereby facilitating separation of the products and unreacted reactants from the catalyst residue. After exiting the reactor the gaseous product stream is cooled sufficiently to condense most of the butyraldehyde product. The noncondensed gas stream exiting the reactor, which include hydrogen, carbon monoxide, lower alkanes, including propylene and propane, and some of the butyraldehyde is conventionally recycled to the reactor. A small part of the recycle stream is purged from the system to prevent buildup of nonreactive components, such as the lower alkanes. The condensate is then stripped, preferably with steam, to remove residual volatiles (mostly propylene and propane) from the product stream. After cooling the gaseous stripper effluent to condense the stream, the volatiles stream is recycled to the reactor.

A significant disadvantage encountered in the above-described process results from the fact that industrial grade propylene usually contains small amounts, for example up to about 10% by volume, propane. Since propane is not generally affected by hydroformylation catalysts, the effluent stream usually contains propane, and the amount of propane present in the effluent may be significant, particularly when low purity propylene is used as feed. In such cases a significant volume of the volatiles must be purged to prevent buildup of propane in the system. Unfortunately, some propylene and butyraldehyde are also discharged from the system in the purge stream.

US-A-4 210 426 relates to a two-step process for separating propylene from the gaseous product of a hydroformylation reaction. The first step involves adsorption to separate the gaseous product into a first gas stream consisting of propane and propylene, and a second gas comprising relatively insoluble gases, particularly hydrogen, argon, nitrogen, carbon monoxide and carbon dioxide. The second step comprises separation by fractional distillation of propylene from the first gas stream.

GB-A-1 375 900 discloses that a carbon molecular sieve containing a minor amount of a metallic component, particularly platinum, can be used to separate a mixture of propane and propene.

EP-A-0 147 960 discloses a pressure swing process for selectively adsorbing and recovering an organic gas containing unsaturated linkages from a mixture of gases by passing the mixture over a zeolite ion-exchanged with cuprous ions. The zeolite has a faujasite-type structure and a silicon to aluminium atomic ratio from 1.2-3.

Because of the difficulty of separating propylene from propane, efficient operation of propylene recycle hydroformylation processes is hard to achieve when the propylene feed contains propane as an impurity. Continuous efforts are underway to enhance the efficiency of recycle butyraldehyde hydroformylation processes, including research investigations for improved procedures for separating propane from propylene prior to recycling the propylene to the reactor. The present invention provides such an improved procedure.

According to the present invention there is provided a process for the production of an oxo product from propylene comprising the steps of:

(a) contacting a propylene stream containing propane as an impurity with carbon monoxide and hydrogen in a reaction zone in the presence of a hydroformylation catalyst under conditions which result in the production of a product stream comprising the oxo product, unreacted propylene and propane;

(b) separating a gas stream comprising unreacted propylene and propane from said product stream;

(c) selectively adsorbing propylene from said gas stream by passing said gas stream through an adsorption zone containing an adsorbent which selectively adsorbs propylene;

(d) regenerating said adsorbent, thereby producing a propylene-enriched gas stream; and

(e) recycling at least part of said propylene-enriched gas stream to said reaction zone.

In one example of a process according to the invention, propylene which contains propane as an impurity, hydrogen and carbon monoxide are introduced into a reaction vessel and contacted therein with a hydroformylation catalyst, which may also function as a hydrogenation catalyst, thereby producing a gaseous product stream comprising mixed butyraldehydes (ie. n-butyraldehyde and i-butyraldehyde) or mixed butyraldehydes and n-butyl alcohol, unreacted propylene, propane and, usually, unreacted carbon monoxide and hydrogen. The product stream is then cooled in a product condenser to produce a condensate which contains most of the butyraldehydes or mixed butyraldehydes and n-butyl alcohol, and some propylene and propane. In addition to the condensate, a gas stream comprising the noncondensable components of the product stream, i.e. carbon monoxide and hydrogen, and some propylene, propane and butyraldehydes is produced. The condensate is next flashed and/or stripped, preferably with steam, thereby evaporating propylene and propane from the condensate. The remaining portion of the condensate, now comprising substantially butyraldehydes or mixed butyraldehydes and n-butyl alcohol, and perhaps small amounts of heavier byproducts, is sent to product recovery for further purification. The vapour phase, which contains propylene, propane and steam, if steam stripping is employed, is cooled to condense the steam, thereby producing a gas stream rich in propylene and propane.

The gas streams from the product condenser and the flash chamber or steam condenser in the above example can be treated in a number of ways. Firstly, all or part of the gas stream from the product condenser and all or part of the gas stream from the flash chamber/steam condenser are subjected to adsorption, thereby adsorbing propylene from the gas stream. The nonadsorbed propane-containing component is discharged from the system and the adsorbed propylene-rich component is recycled to the reactor.

Secondly, the entire gas stream from the steam condenser is recycled to the reactor and all or part of the gas stream from the product condenser is subjected to adsorption to adsorb propylene from this gas stream. The nonadsorbed propane-containing component is discharged from the system and the adsorbed propylene-rich component is recycled to the reactor.

Thirdly, and most preferably, the entire gas stream from the product condenser is recycled to the reactor and all or pad of the gas stream from the flash chamber/steam condenser is subjected to adsorption to adsorb propylene from this gas stream. The nonadsorbed propane-rich component is discharged from the system and the adsorbed propylene-rich component is recycled to the reactor.

In the adsorption system pad or all of the propylene in the gas stream is separated from the propane by pressure swing adsorption or by temperature swing adsorption in one or more adsorption vessels containing beds of adsorbent which selectively adsorb alkenes from gas mixtures containing alkenes and alkanes. The adsorption process is operated under conditions which result in the production of an adsorbed stream enriched in propylene and a non-adsorbed product stream enriched in propane, and is preferably operated to retain substantially all of the unreacted propylene in the product gas stream and reject most of the propane in the stream. The propylene-enriched gas stream obtained upon desorption of the adsorption beds is recycled to the reaction vessel.

In another example of a process according to the invention a propylene-propane gas mixture is subjected to pressure swing adsorption or temperature swing adsorption in beds of adsorbent which selectively adsorb alkenes from gas mixtures containing alkenes and alkanes, as described above, to produce a propylene-enriched stream. The propylene-enriched stream and hydrogen and carbon monoxide are introduced into the reaction vessel and contacted therein with a hydroformylation catalyst, which may also function as a hydrogenation catalyst, thereby producing the gaseous product stream comprising butyraldehydes (n-butyraldehyde and iso-butyraldehyde) or butyraldehydes-n-butyl alcohol mixture, unreacted propylene, propane and perhaps carbon monoxide and hydrogen. The product stream is then cooled in a product condenser to produce a condensate containing most of the butyraldehydes or butyraldehydes-n-butyl alcohol mixture, and some propylene and propane, and a gas stream comprising of carbon monoxide and hydrogen and some propylene, propane and butyraldehydes. The condensate is flashed and/or stripped with steam, as described above, thereby evaporating propylene and propane from the condensate. The condensate, now comprised substantially of butyraldehydes or mixed butyraldehydes and n-butyl alcohol, and perhaps small amounts of heavier byproducts, is sent to product recovery for further purification. The vapour phase, now rich in propylene and propane and perhaps containing steam, is cooled to condense the steam (if steam stripping is employed).

In this example, the gas stream from the product condenser and the vapour phase from the flash chamber and/or steam stripper are also treated according to one of the three recycle-adsorption aspects described above.

The adsorption step of the above-described examples of the invention is typically carried out at a temperature in the range of about 0°C to about 250°C, and is preferably carried out at a temperature of at least about 50°C. The adsorption step is generally carried out at an absolute pressure in the range of about 0.2 to 100 bar, and is preferably carried out carried out at an absolute pressure of about 1 to 50 bar.

In preferred examples of the invention, the adsorbent is type 4A zeolite.

In other preferred examples of the invention the adsorption bed regeneration step is effected by vacuum means or by purging the bed with one or more of an inert gas, the nonadsorbed gas product from the adsorption system or the adsorbed product gas from the adsorption system, or by combinations of vacuum and purge regeneration; and

EP 0 648 730 B1

bed repressurisation is effected using the propylene-enriched desorbed gas from the adsorption system.

The process and apparatus according to the invention will now be described by way of example with reference to the accompanying drawings, in which:

Figure 1 illustrates, in a block diagram, one embodiment of a system for producing butyraldehyde in accordance with the present invention.

Figure 2 illustrates, in a block diagram, an alternative embodiment of the system illustrated in Figure 1.

In the accompanying drawings the same reference letters or numerals are used to designate the same or similar pieces of equipment in different figures. Auxiliary equipment, including compressors, heat exchangers and valves, not necessary for an understanding of the invention, have been omitted from the drawings to simplify discussion of the invention.

The process of the invention can be used to produce oxo products, such as butyraldehyde, butyl alcohol and butyric acid by reacting propylene with carbon monoxide and hydrogen in the presence of an appropriate catalyst. For purposes of illustration however, the invention will be described in particular as it applies to the manufacture of butyraldehyde.

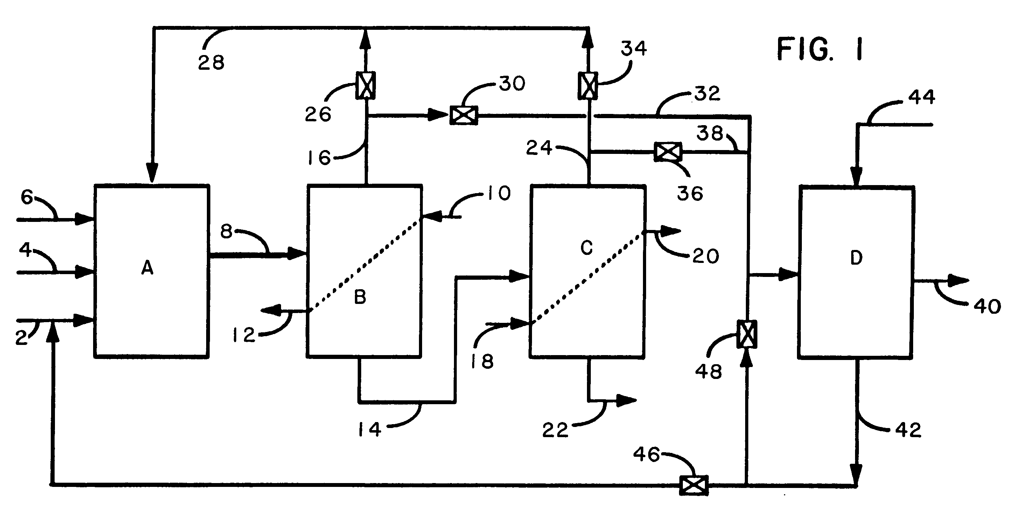

In the system of Figure 1 unit A is a reaction vessel, unit B is a condenser, unit C is a butyraldehyde product recovery unit and D is a propylene separator. Considering Figure 1 in greater detail, a feed stream comprising at least 90 weight percent and preferably at least 95 weight percent propylene, the balance being substantially propane, is introduced into reactor A through line 2. Carbon monoxide and hydrogen are introduced into reactor A through feed lines 4 and 6, respectively. If desired the carbon monoxide and hydrogen may be introduced together, which is convenient when these components comprise a syngas. The catalyst and any other desired additives may be introduced into reactor A either with one of the feed streams or separately through feed lines not shown in Figure 1. The predominant reaction taking place in reactor A proceeds according to the equation:

$$CH_3\text{-}CH = CH_2 + CO + H_2 \leftrightharpoons CH_3\text{-}CH_2\text{-}CH_2\text{-}CHO$$

Reactor A may be any conventional reactor in which the hydroformylation of propylene is carried out, either on a batch or a continuous basis. The reactor is generally provided with stirring means and cooling means to ensure a uniform and controlled reaction. The reactants contact the catalyst in reactor A at a suitable temperature and pressure and a portion of the propylene is hydroformylated to butyraldehydes. The details of the hydroformylation reaction are well known. Typically, the reaction is conducted at temperatures in the range of about 100 to about 175°C, and at pressures in the range about 10 to about 20 atmospheres. Hydroformylation catalysts suitable for use in the reaction include rhodium-triphenylphosphine complex and rhodium-trisulfophenylphosphine complex. Typical hydroformylation processes are the Union Carbide/Davy-Mckee/Johnson-Matthey process using an oil-soluble rhodium-triphenylphosphine catalyst complex, and the Ruhrchemie/Rhone-Poulenc process using a water-soluble rhodium-trisulfophenylphosphine catalyst complex. If it is desired to produce n-butyl alcohol, the alcohol, together with butyraldehyde, is produced by the reaction of propylene, carbon monoxide and hydrogen in the presence of a cobalt hydrocarbonyl catalyst. These processes are well known.

Due to the highly exothermic nature of the hydroformylation reaction, the butyraldehyde product, unreacted propylene, carbon monoxide and hydrogen, and the propane impurity that entered the reactor with the propylene feed stream are all gaseous and thus can be easily separated from the catalyst residue and solvent present in the reactor. These gaseous components leave reactor A through line 8 and are transported to product condenser B.

Product condenser B can be any suitable condenser, and it is generally of the vertical tube and shell design. A coolant, such as cold water, enters condenser B through line 10, passes through the tubes of the condenser and exits condenser B through line 12. The hot gaseous stream entering condenser B through line 8 contacts the outside surfaces of the heat exchange tubes and most of the higher boiling components of the gas stream (butyraldehydes or mixed butyraldehydes-n-butyl alcohol and higher molecular weight byproducts) and some propylene and propane condense and drop to the bottom of the condenser and are removed therefrom through line 14. A noncondensed gas stream, containing the permanent gases, including carbon monoxide, and hydrogen, some propylene and propane and some of the higher boiling components mentioned above, rises to the top of product condenser B and leaves the condenser line 16.

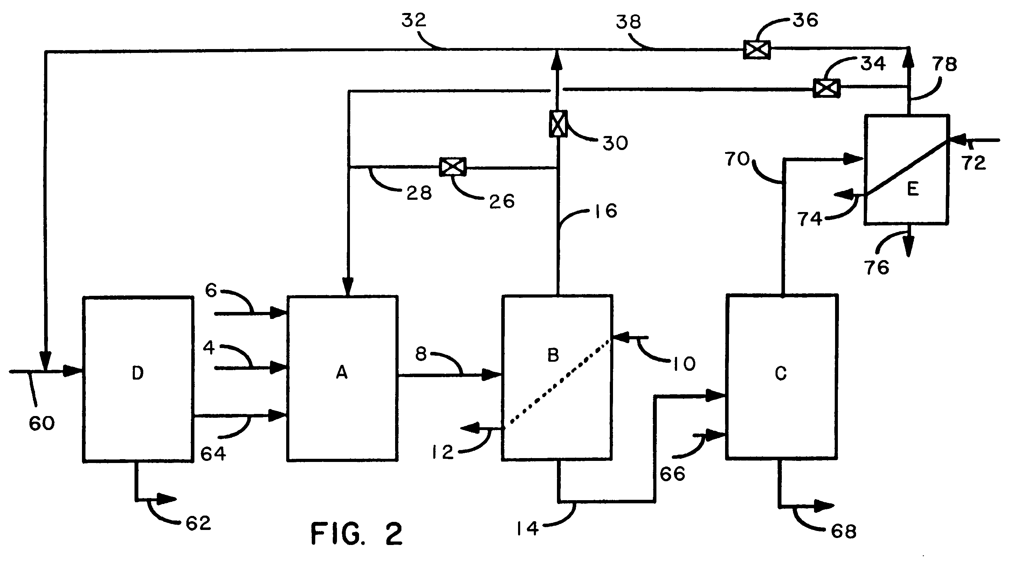

The condensate stream leaving condenser B through line 14 next enters product recovery unit C. Unit C may be a flash chamber or a stripping vessel or it may comprise both a flash chamber and a stripping vessel. When Unit C is a flash chamber, separation of the volatile components from the remaining contents of the reactor effluent is accomplished by heating the contents in unit C, preferably at reduced pressure. The heating can be provided by, for example, electric or steam heating means (not shown). When unit C is a stripper the volatile components in the stream being

4

treated are evaporated by directly passing a hot fluid, such as steam or heated nitrogen through the contents of the stripper, preferably while stirring the contents. It is preferable to use steam as the stripping gas since it can be easily separated from the gaseous effluent from the stripper by cooling the effluent and condensing the steam. In the system illustrated in Figure 1 unit C represents a flash chamber and in the Figure 2 system, described in detail below, unit C represents a steam stripper.

Unit C is typically equipped with agitating means (not shown). The contents of unit C are heated indirectly by steam which enters unit C through line 18, condenses in the heating coils in unit C and exits this unit as steam condensate through line 20. Product condensate from condenser B passes through line 14 and into unit C wherein it is heated sufficiently to evaporate the propylene and propane contained in the condensate with minimal evaporation of the butyraldehyde product. The butyraldehyde product leaves flash chamber C through line 22 and is sent to downstream facilities for product purification and further processing. The propylene-propane gas mixture passes out of unit C through line 24.

Valve 26 controls flow of gas through line 16, which, together with line 28, affords recycle of gas from condenser B to reactor A. Valve 30, located in line 32, controls flow of gas from condenser B to separator D. Valve 34, located in line 24, controls flow of recycle gas from flash chamber C to reactor A via lines 24 and 28. Valve 36, located in line 38 controls flow of gas from flash chamber C to separator D.

As mentioned above, separator D is a pressure swing adsorption system or a temperature swing adsorption system. It may comprise a single adsorption bed or a battery of beds arranged in series or parallel or in combinations of these. In preferred plants separator D comprises two or more adsorbent beds cycled out of phase to provide a pseudo-continuous recycle of unreacted propylene to reactor A. Preferred plants comprise two or more beds operated in a cyclic process comprising adsorption at a relatively high temperature and pressure and desorption or bed regeneration at a relatively low pressure or vacuum, in the case of pressure swing adsorption, and at a temperature higher than the adsorption temperature, in the case of temperature swing adsorption.

The function of separator D is to adsorb unreacted propylene from the feed streams to this unit, the compositions of which will vary depending upon which stream or streams are sent to this unit, but which generally contain unreacted propylene and propane, other light components and possibly some product in gaseous form (and the stripping gas, when this gas is noncondensable). As the gaseous effluent from unit C passes through separator D, substantially all of the unreacted propylene is adsorbed by the propylene-selective adsorbent contained therein. The nonadsorbed gases leave separator D through waste gas discharge line 40. When the unreacted propylene front reaches a predetermined point in separator D, the flow of feed to the particular adsorption unit or units in service is terminated and the regeneration phase of the cycle is begun.

The method or regeneration of the adsorption beds depends upon the type of adsorption process employed. In the case of pressure swing adsorption, the regeneration phase generally includes a countercurrent depressurization step during which the beds are vented countercurrently until they attain atmospheric pressure. If desired the beds may be further depressurized by evacuation to subatmospheric pressure by means of a vacuum inducing device, such as a vacuum pump (not shown). In either case the propylene desorbed from the beds is recycled to reactor A via line 42.

In some cases, in addition to the countercurrent depressurization step(s), it may be desirable to purge the bed with an inert gas or one of the gas streams exiting separator D. In this event the purge step is usually initiated towards the end of the countercurrent depressurization step, or subsequent thereto. During this step, a nonadsorbable purge gas is introduced into separator D via line 44 and passed countercurrently through the adsorbent beds, thereby forcing desorbed propylene out of separator D through line 42. The purge gas may be nonadsorbed product gas exiting separator D through line 40 or a nonadsorbable gas obtained from a different source, such as an inert permanent gas like nitrogen.

When the system of Figure 1 is operated in accordance with the first aspect of the first embodiment mentioned above, valves 30 and 36 are open and valves 26 and 34 may be open or closed. In this aspect some or all of the gases exiting condenser B and flash chamber C are transported to separator D through lines 32 and 38, respectively. If all of the gases from condenser B and flash chamber C are sent to separator D, then both valves 26 and 34 are closed. However if only part of the gas from condenser B is sent to separator D and the remainder is recycled to reactor A, then valve 26 will be open. Similarly if only part of the gas from flash chamber C is sent to separator D and the remainder is recycled to reactor A, then valve 34 will be open.

When the system of Figure 1 is operated in accordance with the second aspect mentioned above, valves 30 and 34 will be open and valve 36 will be closed. Valve 26 may be closed or open depending on whether all or only part of the gas from flash chamber C is sent to separator D.

When the system of Figure 1 is operated in accordance with the third aspect mentioned above, valves 26 and 36 will be open and valve 30 will be closed. Valve 34 may be closed or open depending on whether all or only part of the gas from flash chamber C is sent to separator D. This is the preferred aspect of the embodiment of Figure 1, since the gas stream processed in separator D will comprise mostly propylene and propane. When the system is operated efficiently this alternative will effectively prevent the buildup of propane in the system and permit conversion of substantially all of the propylene entering the system to butyraldehyde and/or n-butyl alcohol.

In an alternative mode of operation of the system of Figure 1 the propylene desorbed from separator D during the countercurrent depressurization step(s) is recycled through line 42 and valve 46 and back to reactor A, and all or a portion of the purge gas and propylene desorbed from the bed during the purge step is recycled to separator D for reprocessing through the adsorption system. This is accomplished by keeping valve 46 open and valve 48 closed during at least part of the countercurrent depressurization step, and closing valve 46 and opening valve 48 at the point during that part of the purge step when it is desired to recycle the purge gas-propylene mixture directly to the feed inlet of separator D. The advantage of this embodiment is that it permits the amount of purge gas that is recycled to the reactor to be minimized.

The adsorption cycle may contain steps other than the fundamental steps of adsorption and regeneration. For example, it may be advantageous to depressurize the adsorption bed in multiple steps, with the first depressurization product being used to partially pressurize another bed in the adsorption system. This will further reduce the amount of gaseous impurities recycled to reactor A. It may also be desirable to include a cocurrent purge step between the adsorption phase and the regeneration phase. The cocurrent purge is effected by terminating the flow of feed gas into separator D and passing high purity propylene cocurrently into the adsorption bed at adsorption pressure. This has the effect of forcing nonadsorbed gas in the void spaces in separator D toward the nonadsorbed gas outlet, thereby ensuring that the propylene produced during the countercurrent depressurization will be of high purity. The high purity propylene used for the cocurrent purge can be obtained from an intermediate storage facility in line 42 (not shown), when separator D comprises a single adsorber; or from another adsorber that is in the adsorption phase, when separator D comprises multiple adsorbers arranged in parallel and operated out of phase; or from propylene feed line 2.

The adsorbent in separator D is a type A zeolite, preferably type 4A zeolite.

Type 4A zeolite, i.e. the sodium form of type A zeolite, has an apparent pore size of about 3.6 to 4 Angstrom units. This adsorbent provides enhanced selectivity and capacity in adsorbing ethylene from ethylene-ethane mixtures and propylene from propylene-propane mixtures at elevated temperatures. This adsorbent is most effective for use in the invention when it is substantially unmodified, i.e. when it has only sodium ions as its exchangeable cations. However, certain properties of the adsorbent, such as thermal and light stability, may be improved by partly exchanging some of the sodium ions with other cations. Accordingly. it is within the scope of the preferred embodiment of the invention to use a type 4A zeolite in which some of the sodium ions attached to the adsorbent are replaced with other metal ions, provided that the percentage of ions exchanged is not so great that the adsorbent loses its type 4A character. Among the properties that define type 4A character are the ability of the adsorbent to selectively adsorb ethylene from ethylene-ethane mixtures and propylene from propylene-propane gas mixtures at elevated temperatures, and to accomplish this result without causing significant oligomerization or polymerization of the alkenes present in the mixtures. In general, it has been determined that up to about 25 percent (on an equivalent basis) of the sodium ions in 4A zeolite can be replaced by ion exchange with other cations without divesting the adsorbent of its type 4A character. Cations that may be ion exchanged with the 4A zeolite used in the alkene-alkane separation include, among others, potassium, calcium, magnesium, strontium, zinc, cobalt, silver, copper, manganese, cadmium, aluminum, cerium, etc. When exchanging other cations for sodium ions it is preferred that less than about 10 percent of the sodium ions (on an equivalent basis) be replaced with such other cations. The replacement of sodium ions may modify the properties of the adsorbent. For example, substituting some of the sodium ions with other cations may improve the stability of the adsorbent.

The temperature at which the adsorption step is carried out depends upon a number of factors, such as the particular adsorbent being used, e.g. unmodified 4A zeolite, or a particular metal-exchanged 4A zeolite which selectively adsorbs alkenes from alkene-alkane mixtures, and the pressure at which the adsorption is carried out. In general, the adsorption step is carried out at a minimum temperature of about 50°C and is preferably carried out at a temperature of at least about 70°C. The upper temperature limit at which the adsorption step in unit A is carried out is determined mostly by economics. In general the adsorption step can be carried out at a temperature below the temperature at which the alkene undergoes chemical reaction, such as polymerization. When unmodified 4A zeolite is used as the adsorbent the reaction is generally carried out at or below 250°C, and is preferably carried out at a temperature at or below 200°C.

The pressures at which the adsorption and regeneration steps of the pressure swing adsorption embodiment of the invention are carried out are not critical, and in general, these steps can be carried out at any of the usual pressures employed for gas adsorption processes, with the limitation, of course, that the adsorption step be carried out at a pressure greater than the regeneration step pressure. Typically, when the adsorption process is pressure swing adsorption the absolute pressure during the adsorption step will range generally from about 0.2 to about 20 atmospheres, and preferably from about 1 to 10 atmospheres, and during the regeneration step will range from about 20 millibar to about 1 atmosphere or more. When the adsorption process is temperature swing adoption the pressure during both adsorption and desorption is desirably atmospheric or near atmospheric.

Figure 2 illustrates an alternative embodiment of the invention described with reference to Figure 1. In the Figure 2 embodiment separator D is positioned upstream of reactor A. Except for the fact that separator D of Figure 2 may be larger than separator D of Figure 1, equipment units A, B and D of Figures 1 and 2 are substantially identical. As mentioned above, unit C of Figure 2 represents a gas stripping unit. Unit E is a steam condenser.

In practising the process of the invention in the system of Figure 2, a feed stream comprised substantially of propylene, but containing propane as an impurity, is introduced into separator D through line 60. The feed stream is subjected to pressure swing adsorption or temperature swing adsorption in separator D, as described above. Nonadsorbed propane-enriched product is discharged from separator D through line 62 and desorbed propylene-enriched product is recovered from unit D through line 64. The propylene-enriched product next enters reactor A wherein the propylene reacts with the carbon monoxide and hydrogen entering reactor A through lines 4 and 6, respectively, to form butyraldehyde and/or n-butyl alcohol under the conditions set forth above. The reaction product is discharged from reactor A through line 8 and it next enters condenser B, wherein noncondensed gases are separated from the condensed reaction product, as described above. These gases are removed from unit B through line 16 and recycled to reactor A or transported to separator D, as described below. The condensate from condenser B is forwarded to stripper C through line 14. In unit C the condensate is stripped with steam, which enters this unit through line 66, thereby vaporizing substantially all of the propylene and propane contained in the condensate. The degassed condensate leaves stripper C through line 68 and is sent to downstream units for product recovery and further processing. The stripped propylene and propane and the steam exit unit C through line 70 and next enter steam condenser E. The steam in the vapour stream is condensed in condenser E by a cooling medium, such as water, which enters unit E through line 72 and exits this unit through line 74. The steam condensate is removed from condenser E through line 76 and returned to the steam regenerating means, and the propylene-propane gas stream passes out of unit E through line 78. All or a portion of the recovered propylene-propane gas stream is recycled to separator D or reactor A, as described below.

When the system of Figure 1 is operated in the first way mentioned above, valves 30 and 36 are open and valves 26 and 34 may be open or closed. In this aspect some or all of the gases exiting condenser B and steam condenser E are transported to separator D through lines 16, 38 and 32. If all of the gases from condenser B and steam condenser E are sent to separator D, then both valves 26 and 34 are closed. However if only part of the gas from condenser B is sent to separator D and the remainder is recycled to reactor A, then valve 26 will be open. Similarly if only part of the gas from steam condenser E is sent to separator D and the remainder is recycled to reactor A, then valve 34 will be open.

When the system of Figure 2 is operated in the second way mentioned above, valves 30 and 34 will be open and valve 36 will be closed. Valve 26 may be closed or open depending on whether all or only part of the gas from flash chamber C is sent to separator D.

When the system of Figure 2 is operated in the third way mentioned above, valves 26 and 36 will be open and valve 30 will be closed. Valve 34 may be closed or open depending on whether all or only part of the gas from flash chamber C is sent to separator D. As was the case with the embodiment of Figure 1, this is the preferred aspect of the embodiment of Figure 2, since the gas stream processed in separator D will comprise mostly propylene and propane.

It will be appreciated that it is within the scope of the present invention to utilize conventional equipment to monitor and automatically regulate the flow of gases within the system so that it can be fully automated to run continuously in an efficient manner.

Important advantages of the invention are that it permits use of a less pure propylene feed stream than was formerly possible, and the process can be run at a relatively low per pass conversion of the propylene feed to the desired product, thereby achieving substantially improved selectivity. It will be appreciated that a system that achieves enhanced selectivity, and hence increased overall yield of a desired product, is highly beneficial.

The invention is further illustrated by the following example in which, unless otherwise indicated, parts, percentages and ratios are on a volume basis.

EXAMPLE I

This example is a hypothetical example depicting the production of butyraldehyde using as feed to the reactor a propylene stream containing propane as an impurity, carbon monoxide and hydrogen. The proposed propylene feed stream is comprised of 95.0% propylene and 5.0% propane. The ratio of carbon monoxide to propylene feed is 0.46 to 1 and the ratio of hydrogen to propylene is 2.0 to 1. The proposed reaction system is similar to the system illustrated in Figure 1 except that the reactor effluent is separated into a gas phase and a condensate phase in a product condenser, and part of the gas phase is recycled to the butyraldehyde reactor and the remainder is subjected to a pressure swing adsorption process using zeolite type 4A as adsorbent. The butyraldehyde reactor is to be operated at a temperature of 130°C and an absolute pressure of 14 atmospheres. The adsorption process is to be carried out at an adsorption pressure of 0.7 bar and a bed regeneration pressure of 300 mbar. Impurities including iso-butyraldehyde, aldol and higher aldehydes ("trimer" and "tetramer" in the Table) are formed in the product as a result of by-reactions.

The results of the run are recorded in the Table. In the Table, stream 1 is the fresh feed flow to the system;

stream 2 is the combined total of the fresh feed flow and the flows of all recycle streams to the reactor; stream 3 is the flow of reactor effluent to the product condenser; stream 4 is the flow of condensed product from the product condenser; stream 5 is the total flow of gas phase from the product condenser; stream 6 is the flow of gas recycled from the product condenser directly to the reactor; stream 7 is the flow of gas from the product condenser to the pressure swing adsorption system; stream 8 is the flow of desorbed recycle gas from the pressure swing adsorption system to the reactor; and stream 9 is the flow of waste gas from the pressure swing adsorption system.

**TABLE I**

| COMPONENT | SEL. | STREAM | | | | | | | | |
|---|---|---|---|---|---|---|---|---|---|---|
| | | 1 moles | 2 moles | 3 moles | 4 moles | 5 moles | 6 moles | 7 moles | 8 moles | 9 moles |
| Propylene | | 112.939 | 719.647 | 609.757 | 0.000 | 609.757 | 548.781 | 60.976 | 57.927 | 3.049 |
| Propane | 0.004 | 5.944 | 79.736 | 80.208 | 0.000 | 80.208 | 72.188 | 8.021 | 1.604 | 6.417 |
| CO | | 130.198 | 328.159 | 218.741 | 0.000 | 218.741 | 196.867 | 21.874 | 1.094 | 20.780 |
| $H_2$ | | 235.093 | 1432.314 | 1322.896 | 0.000 | 1322.896 | 1190.606 | 132.290 | 6.614 | 125.675 |
| Methane | | 7.946 | 88.290 | 88.290 | 0.000 | 88.290 | 79.461 | 8.829 | 0.883 | 7.946 |
| i-Butyraldehyde | 0.076 | 0.000 | 2.659 | 8.352 | 5.679 | 2.673 | 2.405 | 0.267 | 0.254 | 0.013 |
| n-Butyraldehyde | 0.910 | 0.000 | 24.278 | 100.000 | 75.600 | 24.400 | 21.960 | 2.440 | 2.318 | 0.122 |
| Trimer | 0.005 | 0.000 | 0.000 | 0.168 | 0.168 | 0.000 | 0.000 | 0.000 | 0.000 | 0.000 |
| Tetramer | 0.000 | 0.000 | 0.000 | 0.005 | 0.005 | 0.000 | 0.000 | 0.000 | 0.000 | 0.000 |
| Aldol | 0.005 | 0.000 | 0.000 | 0.269 | 0.269 | 0.000 | 0.000 | 0.000 | 0.000 | 0.000 |
| Total | 1.000 | 492.120 | 2675.083 | 2428.688 | 81.722 | 2346.966 | 2112.269 | 234.697 | 70.694 | 164.003 |

EP 0 648 730 B1

**Claims**

1. A process for the production of an oxo product from propylene comprising the steps of:

   (a) contacting a propylene stream containing propane as an impurity with carbon monoxide and hydrogen in a reaction zone in the presence of a hydroformylation catalyst under conditions which result in the production of a product stream comprising butyraldehyde (or other oxo product), unreacted carbon monoxide, hydrogen, propylene and propane;

   (b) condensing the product stream so as to obtain a condensate comprising most of the butyraldehyde and some propylene and propane, and a first non-condensed gas stream comprising carbon monoxide, hydrogen and some propane and propylene;

   (c) separating a second gas stream comprising unreacted propylene and propane from said condensate;

   (d) passing through an adsorption zone containing an adsorbent which selectively adsorbs propylene

      (i) all or part of the first gas stream and all or part of the second gas stream; or

      (ii) all or part of the first gas stream, the entire second gas stream being recycled to step (a), or

      (iii) all or part of the second gas stream, the entire first gas stream being recycled to step (a)
      and thereby selectively adsorbing propylene from the respective gas stream or gas streams;

   (e) regenerating said adsorbent, thereby producing a propylene-enriched gas stream; and

   (f) recycling at least part of said propylene-enriched gas stream to said reaction zone, and recycling to said reaction zone any of said first and second gas streams not passed to said adsorption zone;
   wherein the adsorbent is a type (A) zeolite.

2. A process according to Claim 1, wherein step (c) is carried out by flashing unreacted propylene and propane from said condensate, by stripping unreacted propylene and propane from said condensate, or by first flashing unreacted propylene and propane from said condensate and then stripping unreacted propylene and propane from said condensate.

3. A process according to claim 1 or claim 2, wherein fresh gas mixture comprising propylene and propane is also separated in said adsorption zone.

4. A process according to any one of the preceding claims, wherein the adsorption step is conducted at a temperature above 50°C.

5. A process according to Claim 4, wherein the adsorption step is conducted at a temperature in the range of 50 to 250°C.

6. A process according to any one of the preceding claims, wherein the adsorbent is type 4A zeolite.

7. A process according to Claim 6, wherein the said adsorbent contains exchangeable cations other than sodium ions, but at a level insufficient to divest the adsorbent of its type 4A character.

8. A process according to any one of the preceding claims, wherein the adsorption step and regeneration step are comprised in a pressure swing adsorption cycle.

**Patentansprüche**

1. Verfahren zur Herstellung eines Oxo-Produkts aus Propylen, mit folgenden Schritten:

   a) In-Berührung-Bringen eines Propylenstroms, der Propan als Verunreinigung enthält, mit Kohlenmonoxyd und Wasserstoff in einer Reaktionszone in Anwesenheit eines Hydroformylierungskatalysators unter Bedingungen, die zur Erzeugung eines Produktstroms führen, der Butyraldehyd (oder ein anderes Oxo-Produkt), nichtreagiertes Kohlenmonoxyd, Wasserstoff, Propylen und Propan enthält,

b) Kondensieren des Produktstroms, um so ein Kondensat zu erhalten, welches den größten Teil des Butyraldehyds und einiges Propylen und Propan enthält, und einen ersten nichtkondensierten Gasstrom zu erhalten, der Kohlenmonoxyd, Wasserstoff und ein Teil des Propans und Propylens enthält,

c) Abtrennen eines zweiten Gasstroms, der nichtreagiertes Propylen und Propan enthält, aus dem Kondensat,

d) Hindurchleiten durch eine Absorptionszone mit selektiv Propylen adsorbierendem Adsorptionsmittel

   i) den gesamten ersten Gasstrom oder einen Teil hiervon und den gesamten zweiten Gasstrom oder einen Teil hiervon, oder

   ii) den gesamten ersten Gasstrom oder einen Teil hiervon, während der gesamte zweite Gasstrom zum Schritt a) rezirkuliert wird, oder

   iii) den gesamten zweiten Gasstrom oder einen Teil hiervon, während der gesamte erste Gasstrom zum Schritt a) rezirkuliert wird,
   und dadurch selektives Adsorbieren von Propylen aus dem jeweiligen Gasstrom bzw. den Gasströmen,

e) Regenerieren des Adsorptionsmittels, wodurch ein Propylenangereicherter Gasstrom erzeugt wird, und

f) Rezirkulieren mindestens eines Teils des Propylen-angereicherten Gasstroms in die Reaktionszone, und Rezirkulieren irgendwelcher nicht zur Adsorptionszone geleiteter Teile des ersten und des zweiten Gasstroms, wobei das Adsorptionsmittel ein Typ-A-Zeolith ist.

2. Verfahren nach Anspruch 1, wobei der Schritt c) durch Entspannungsverdampfung von nichtreagiertem Propylen und Propan aus dem Kondensat, durch Strippen von nichtreagiertem Propylen und Propan aus dem Kondensat, oder durch erst stattfindendes Entspannungsverdampfen von nicht reagiertem Propylen und Propan aus dem Kondensat und anschließendes Strippen von nichtreagiertem Propylen und Propan aus dem Kondensat ausgeführt wird.

3. Verfahren nach Anspruch 1 oder 2, wobei frisches Gasgemisch, das Propylen und Propan enthält, ebenfalls in der Adsorptionszone getrennt wird.

4. Verfahren nach einem der vorhergehenden Ansprüche, wobei der Adsorptionsschritt bei einer Temperatur oberhalb 50°C ausgeführt wird.

5. Verfahren nach Anspruch 4, wobei der Adsorptionsschritt bei einer Temperatur im Bereich von 50 bis 250°C ausgeführt wird.

6. Verfahren nach einem der vorhergehenden Ansprüche, wobei das Adsorptionsmittel ein Typ-4A-Zeolith ist.

7. Verfahren nach Anspruch 6, wobei das Adsorptionsmittel andere austauschbare Kationen als Natriumionen enthält, aber in einer unzureichenden liege, um den Typ-4A-Charakter zu verlieren.

8. Verfahren nach einem der vorhergehenden Ansprüche, wobei der Adsorptionsschritt und der Regenerationsschritt in einem Druckwechsel-Adsorptionszyklus erfolgen.

**Revendications**

1. Procédé de préparation de composés oxo à partir de propylène, comprenant les étapes consistant à :

   (a) mettre en contact un flux de propylène contenant en impureté du propane, avec du monoxyde de carbone et de l'hydrogène dans une zone de réaction en présence d'un catalyseur d'hydroformylation sous des conditions qui aient pour résultat la production d'un flux de produits comprenant du butyraldéhyde (ou un autre produit oxo), du monoxyde de carbone n'ayant pas réagi, de l'hydrogène, du propylène et du propane ;

(b) condenser le flux de produits afin d'obtenir un condensat comprenant la majorité du butyraldéhyde et un peu de propylène et de propane, et un premier flux de gaz non condensé comprenant du monoxyde de carbone, de l'hydrogène et un peu de propane et de propylène ;

(c) séparer un second flux de gaz comprenant du propane et du propylène n'ayant pas réagi dudit condensat ;

(d) faire passer dans une zone d'adsorption contenant un adsorbant qui adsorbe sélectivement le propylène :

    (i) tout ou partie du premier flux de gaz et tout ou partie du second flux de gaz ; ou
    (ii) tout ou partie du premier flux de gaz, la totalité du second flux de gaz étant recyclée vers l'étape (a), ou
    (iii) tout ou partie du second flux de gaz, la totalité du premier flux de gaz étant recyclée vers l'étape (a)
    et ainsi adsorber sélectivement le propylène du flux ou des flux de gaz respectif(s) ;

(e) régénérer ledit adsorbant, produisant ainsi un flux de gaz enrichi en propylène ; et

(f) recycler au moins une partie dudit flux de gaz enrichi en propylène vers ladite zone de réaction, et recycler vers ladite zone de réaction l'un quelconque desdits premier et second flux de gaz qui n'est pas passé dans ladite zone d'adsorption ;

dans lequel l'adsorbant est une zéolithe de type A.

2. Procédé selon la Revendication 1, dans lequel l'étape (c) est réalisée par libération éclair du propylène et du propane n'ayant pas réagi à partir dudit condensat, par stripage du propylène et du propane n'ayant pas réagi à partir dudit condensat, ou d'abord par libération éclair du propylène et du propane n'ayant pas réagi à partir dudit condensat, suivi du stripage du propylène et du propane n'ayant pas réagi à partir dudit condensat.

3. Procédé selon la Revendication 1 ou la Revendication 2, dans lequel le mélange de gaz frais comprenant le propylène et le propane est également séparé dans ladite zone d'adsorption.

4. Procédé selon l'une quelconque des Revendications précédentes, dans lequel l'étape d'adsorption est réalisée à une température supérieure à 50°C.

5. Procédé selon la Revendication 4, dans lequel l'étape d'adsorption est effectuée à une température comprise entre 50 et 250°C.

6. Procédé selon l'une quelconque des Revendications précédentes, dans lequel l'adsorbant est une zéolithe de type 4A.

7. Procédé selon la Revendication 6, dans lequel ledit adsorbant contient des cations échangeables autres que des ions sodium, mais selon des proportions insuffisantes pour priver l'adsorbant de son caractère de type 4A.

8. Procédé selon l'une quelconque des Revendications précédentes, dans lequel l'étape d'adsorption et l'étape de régénération sont englobées dans un cycle d'adsorption par variation de la pression.

FIG. I

FIG. 2

EP 0 648 730 B1